(19) **Europäisches Patentamt**

**European Patent Office**

**Office européen des brevets**

(11) Veröffentlichungsnummer: **0 337 130**

**A2**

(12) # EUROPÄISCHE PATENTANMELDUNG

(21) Anmeldenummer: **89104409.1**

(51) Int. Cl.⁴ **A45D 24/22** , **A61K 7/06**

(22) Anmeldetag: **13.03.89**

(30) Priorität: **14.12.88 DE 3842006**
**22.03.88 DE 3809498**

(43) Veröffentlichungstag der Anmeldung:
**18.10.89 Patentblatt 89/42**

(84) Benannte Vertragsstaaten:
**ES GR**

(71) Anmelder: **Henkel Kommanditgesellschaft auf Aktien**
**Postfach 1100 Henkelstrasse 67**
**D-4000 Düsseldorf 1(DE)**

(72) Erfinder: **Busch, Peter, Dr.**
**Gottfried-August-Bürger-Strasse 10**
**D-4006 Erkrath(DE)**
Erfinder: **Thiele, Klaus**
**Rügenweg 5**
**D-4018 Langenfeld(DE)**
Erfinder: **Höffkes, Horst, Dr.**
**Carlo-Schmid-Strasse 113**
**D-4000 Düsseldorf-Hellerhof(DE)**

(54) Vorrichtung zur Haarbehandlung.

(57) Mit einer Vorrichtung, bestehend aus einem Spender in Form eines Kammes oder einer Bürste und einer Wirkstoffzubereitung, die mindestens eine haaravivierend oder haarfestigend wirkende Komponente in einer Konzentration von 0,01 bis 20 Gew.-%, bezogen auf die Gesamtmasse der Wirkstoffzubereitung, enthält und bei Raumtemperatur eine Viskosität von 0,9 bis 12 mPas aufweist, ist eine gezielte, das Haar und die Kopfhaut schonende und mit geringem Aufwand durchführbare Haarnachbehandlung möglich.

EP 0 337 130 A2

## Vorrichtung zur Haarbehandlung

Gegenstand der Erfindung sind Vorrichtungen zur Haarbehandlung, bestehend aus einem Spender in Form eines Kammes oder einer Bürste und einer Wirkstoffzubereitung zur Pflege, Avivage oder Festigung des Haares.

Viele Arten der Haarbehandlung, bei denen Wirkstoffe auf das Haar gebracht werden, führen neben den gewünschten Effekten auch zu einer erheblichen Belastung oder Schädigung des Haares. Es ist daher beispielsweise nach einer Dauerwellbehandlung oder Färbung der Haares angebracht, die Haare einer Nachbehandlung zu unterziehen. Die dabei auf des Haar aufgebrachten, avivierend, konditionierend und/oder festigend wirkenden Substanzen sollen einer dauerhaften Schädigung der Haare vorbeugen und diesen weitere gewünschte Eigenschaften wie beispielsweise eine gute Naß- und Trockenkämmbarkeit, eine längere Haltbarkeit der Frisur und einen Schutz vor feuchter Luft verleihen.

Gängige Nachbehandlungen sind Spülungen, bei denen die Wirkstoffe beispielsweise in Form einer Lösung, einer Dispersion, einer Creme oder einer Emulsion auf das Haar aufgebracht werden. Nach einer Einwirkzeit wird das Haar dann gespült und anschließend getrocknet. Solche Nachbehandlungen sind also mit einem erheblichen Aufwand verbunden und führen häufig auch zu einer weiteren, unerwünschten Belastung der Kopfhaut. Weiterhin werden zum Schutz der Frisur vor Feuchtigkeit und zur Verbesserung der Haltbarkeit schnelltrocknende Harzlösungen verwendet, die in der Regel als Haarsprays formuliert werden. Auch hier wäre eine gezieltere Abgabe der Wirkstoffe auf das Haar sowie eine Umgehung der bei der Formulierung von Sprays auftretenden Treibgas-Problematik von Vorteil.

Es besteht daher ein Bedarf nach einem einfachen, die Kopfhaut nicht oder nur wenig belastenden Haarnachbehandlungsverfahren. Weiterhin wäre es vorteilhaft, wenn einzelne, besonders beanspruchte Haarregionen gezielt nachbehandelt werden könnten.

Es ist bekannt, daß mit Hilfe eines Spenders, beispielsweise in Form eines Haarkammes oder einer Haarbürste, das gesamte Haar oder einzelne Haarsträhnen mit einem geringen Aufwand und ohne Beeinträchtigung der Kopfhaut eingefärbt werden können. Solche Spender, die beim Kämmen oder Bürsten der Haare gleichzeitig eine Flüssigkeit, insbesondere ein Haarfärbemittel abgeben, sind beispielsweise in den deutschen Offenlegungsschriften 27 49 074 und 36 22 234 und der deutschen Gebrauchsmusteranmeldung 79 32 856 beschrieben. Ein weiterer Spender ist in der deutschen Patentanmeldung 38 09 498 der Anmelderin beschrieben.

Die Spender bestehen im Prinzip aus einem Vorratsgefäß für die aufzutragende Flüssigkeit, das über Verbindungskanäle mit den Zinken oder Borsten verbunden ist, über die die Flüssigkeit abgegeben wird. Dieses Vorratsgefäß kann auf den Kamm oder die Bürste aufgesetzt oder beispielsweise in deren Stiel oder Rücken integriert sein. Die Spender können sowohl starr sein, als auch gewünschtenfalls mit einem Vibrationsmechanismus ausgestattet sein, der die Abgabe der Flüssigkeit verstärkt.

Es wurde nun gefunden, daß Spender in Kombination mit bestimmten haaravivierenden und/oder festigenden Wirkstoffzubereitungen besonders vorteilhafte Eigenschaften zeigen.

Gegenstand der Erfindung ist somit eine Vorrichtung zur Haarbehandlung, bestehend aus einem Spender in Form eines Kammes oder einer Bürste und einer Wirkstoffzubereitung, die mindestens eine haaravivierend oder haarfestigend wirkende Komponente enthält, dadurch gekennzeichnet, daß die haaravivierenden und/oder haarfestigenden Komponenten in einer Konzentration von 0,01 bis 20 Gew.-%, bezogen auf die Gesamtmasse der Wirkstoffzubereitung, enthalten sind und daß die Wirkstoffzubereitung bei Raumtemperatur eine Viskosität von 0,9 bis 12 mPas aufweist.

Als Spender können Kämme oder Bürsten der oben genannten Gattung verwendet werden. Bevorzugt ist ein Spender gemäß der deutschen Patentanmeldung 38 09 498. Dieser Spender besteht aus einem Speicher für die Wirkstoffzubereitung aus einem ersten porösen Material innerhalb eines Gehäuses und in das erste poröse Material eingesetzten, mit diesem in Kapillarverbindung stehenden Zinken aus einem zweiten porösen Material, wobei die Zinken im Oberflächenbereich ihrer freien Enden mit verschlossenen Poren ausgebildet sind und die Hauptporenrichtung des zweiten porösen Materials der Zinken und des ersten porösen Materials des Speichers im wesentlichen gleichgerichtet parallel zur Zinkenlängsrichtung ist und sich das erste poröse Material des Speichers über ein Mehrfaches der Eindringtiefe der Zinken in Eindringrichtung erstreckt.

Bezüglich der Details dieses Spenders wird ausdrücklich auf den Inhalt der genannten Patentanmeldung Bezug genommen.

Der Spender kann als Einmalprodukt ausgestaltet sein, der nach Erschöpfung des Vorrates an Wirkstoffzubereitung weggeworfen wird. Es ist jedoch bevorzugt, den Spender so zu gestalten, daß eine Nachfüllmöglichkeit für die Wirkstoffzubereitung besteht und die Vorrichtung somit zur dauerhaften Verwendung geeignet ist.

Bevorzugt enthält die Wirkstoffzubereitung Konzentrationen an haaravivierenden und/oder Komponenten von 0,01 bis 10 Gew.-%, insbesondere von 0,01 bis 5 Gew.-%, bezogen auf die Gesamtmasse der Wirkstoffzubereitung.

Die Wirkstoffzubereitung kann beispielsweise als Lösung, Dispersion oder Emulsion formuliert werden. Als Lösungs- oder Dispersionsmittel können beispielsweise Wasser, niedere Alkohole und andere hautverträgliche organische Verbindungen verwendet werden. Im Rahmen der erfindungsgemäßen Lehre ist die Formulierung als wäßrige Lösung oder Emulsion bevorzugt.

Die für den Spender bestimmte Zubereitung muß bestimmte rheologische Eigenschaften aufweisen. Um die Abgabe einer ausreichenden Wirkstoffmenge bei der Anwendung sicherzustellen, darf die Viskosität der Wirkstoffzubereitung nicht zu hoch sein. Andererseits muß die Viskosität einem bestimmten Minimalwert aufweisen, um einem ungewollten und unkontrollierten Ausfließen der Wirkstoffzubereitung vorzubeugen. Die Wirkstoffzubereitungen müssen daher auf eine Viskosität von etwa 0,9 bis 12 mPas bei Raumtemperatur eingestellt werden. Viskositäten von etwa 0,9 bis 6,5 mPas bei Raumtemperatur sind im Rahmen der erfindungsgemäßen Lehre besonders bevorzugt.

Diese Viskositäten können beispielsweise mit einem Kugelfallviskosimeter nach Höppler bestimmt werden.

Es hat sich gleichfalls als vorteilhaft erwiesen, wenn die Wirkstoffzubereitungen eine Oberflächenspannung von 20-70 dyn/cm, insbesondere von 30-40 dyn/cm, aufweisen.

Des weiteren dürfen die in den Zubereitungen verwendeten Komponenten keinen zu großen Dampfdruck aufweisen, der bei einer längeren Lagerung zu einer merklichen Verarmung der Mischung an dieser Komponente und dadurch verursachten Änderungen beispielsweise im rheologischen Verhalten führen könnte.

Wird die Zubereitung in Form einer Lösung formuliert, so müssen die Wirkstoffe in den gewählten Lösungsmitteln ausreichend löslich sein, um die gewünschte Menge auf das Haar aufbringen zu können. Es werden daher bevorzugt haaravivierende und -festigende Komponenten verwendet, die eine Löslichkeit von mindestens 0,1 g/l in dem gewählten Lösungsmittel, insbesondere in Wasser, aufweisen.

Bevorzugte haaravivierende Wirkstoffe sind quaternäre Ammoniumverbindungen.

Geeignete quartäre Ammoniumverbindungen sind beispielsweise Alkyltrimethylammoniumsalze mit 12-22 C-Atomen in der gegebenenfalls substituierten Alkylkette wie z.B. Hexadecyltrimethylammoniumsalze und Ricinolsäurepropylamidotrimethylammoniumsalze, und Dialkyldimethylammoniumsalze mit 2-22 C-Atomen in der gegebenenfalls substituierten Alkylkette wie z.B. 2-Hydroxyhexadecyl-2-hydroxyethyldimethylammoniumsalze, Dodecylethyldimethylammoniumsalze, Stearyl benzyldimethyl-, Dodecylbenzyldimethyl- und andere Alkylbenzyldimethylammoniumsalze, Distearyldimethylammoniumsalze, Lanolinfettsäureamidopropylethyldimethylammoniumsalze, Cetyl-2-hydroxyethyldimethylammoniumsalze, Dikokosacyldimethylammoniumsalze, Dirübacyl-1,1-dimethylethyl-dimethylammoniumsalze, Diölsäureisopropylesterdimethylammoniumsalze, Gluconamidopropyl-2-hydroxyethyl-dimethylammoniumsalze, Minkamidopropyl-2-hydroxyethyl-dimethylammoniumsalze. Weiterhin geeignet sind Polyoxyethylstearylammoniumsalze, Penta(oxyethylen)octadecylammoniumsalze, Bis-talgacylaminoethyl-2-hydroxyethyl-methylammoniumsalze, Kokospentaethoxymethylammoniumsalze, Alkyl-3-acylaminoethylimidazoliniumsalze, Talgalkylamidoethyl-methyl-2-talgalkylimidazoliniumsalze, Bis-oleylamidoethyl-2-oleylimidazoliniumsalze und Alkylamidoethyl-methyl-alkylimidazoliniumsalze. Ebenfalls geeignet sind Verbindungen von Typ der Alkylpyridiniumsalze wie Laurylpyridiniumsalze. Die in den genannten Verbindungen enthaltenen Alkyl- und Acylgruppen haben üblicherweise etwa 12 bis 22 Kohlenstoffatome. Als Gegenionen in diesen Salzen können beispielsweise Halogenidionen, insbesondere Chlorid- und Bromidionen, Lactationen, Methosulfationen, Ethosulfationen, Phosphationen, Hydrogen- und Dihydrogenphosphationen, Saccharinationen und Acetationen fungieren.

Weiterhin können als haaravivierende Komponenten ampholytische und/oder zwitterionische Tenside eingesetzt werden.

Als zwitterionische Tenside werden solche oberflächenaktiven Verbindungen bezeichnet, die im Molekül mindestens eine quartäre Ammoniumgruppe und mindestens eine -COO$^{(-)}$- oder -SO$_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acylaminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethyl-hydroxyethylcarboxymethylglycinat.

Unter ampholytischen Tensiden werden solche oberflächenaktiven Verbindungen verstanden, die außer einer C$_8$-C$_{18}$-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.

Eine bevorzugte Gruppe von haarfestigenden Wirkstoffen stellen filmbildende Polymere dar.

Geeignete Substanzen sind beispielsweise Polyvinylpyrrolidone und deren Co- und Terpolymere mit Vinylacetat und/oder Vinylpropionat (Luviskol®, Nasuna®), Polybutyraldehydacetale (Nasuna®), quartäre Polymere, die durch Reaktion von Polyvinylpyrrolidon mit quaternären Ammoniumverbindungen entstehen (Gafquat®), Mischpolymerisate aus Methylvinylether und Maleinsäureanhydrid (Gantrez®), Copolymere aus Vinylacetat und N-Vinyl-5-methyl-2-oxazolidon ((Devlex®), Mischungen aus Polyvinylpyrrolidon, Acrylatestern und (Meth)acrylsäure (VEM-Harze®, Polyacrylate (Carboset®), carboxylierte Vinylacetat-Terpolymere (Resyn®), amphotere Acrylharze (Amphomer®), Polyglykol-Polyamin-Kondensationsharze (Polyquart®), kationische, quaternierte Cellulosen (Polymer JR®, Celquat®) sowie das polymere Dimethyldiallylammoniumchlorid und dessen Copolymere mit Acrylamid (Merquat®), sofern sie in der Grundlage der Wirkstoffzubereitung, d.h. in der Regel in Wasser, einem verwendbaren organischen Lösungsmittel oder einer entsprechenden wäßrigen Mischung hinreichend löslich sind.

Die erfindungsgemäßen Zubereitungen können organische Lösungsmittel, bevorzugt in Mengen von 0,1 - 10 Gew.-% bezogen auf die Wirkstoffzubereitung, insbesondere an niederen Alkoholen mit 1-4 Kohlenstoffatomen, wie beispielsweise Ethanol, 1-Propanol, Isopropanol, Ethylenglykol, Glycerin oder Butanol enthalten. Ethanol und Isopropanol sind bevorzugte organische Lösungsmittel.

Als weitere Komponenten in den erfindungsgemäßen Wirkstoffzubereitungen können gewünschtenfalls anionische, kationische und/oder nichtionogene Tenside, Ölkomponenten, biogene Wirkstoffe wie Lecithin, Tocopherole, Pflanzenextrakte, Proteine und Proteinhydrolysate, Überfettungsmittel wie Polyolfettsäureester, Riechstoffe sowie Substanzen zur Einstellung des pH-Wertes verwendet werden.

Der pH-Wert der erfindungsgemäßen Wirkstoffzubereitungen liegt zwischen 2 und 10. Bevorzugt ist ein pH-Wert von 5-8, insbesondere von 5-7. Zur Einstellung des gewünschten pH-Wertes können organische Säuren wie beispielsweise Zitronensäure oder Ammoniak verwendet werden. Es kann bevorzugt sein, durch Zugabe entsprechender Salze, z.B. Natriumcitrat, Ammoniumchlorid, Ammoniumsulfat oder Ammoniumcarbonat, bezüglich des pH-Wertes gepufferte Wirkstoffzubereitungen einzustellen.

Weiterhin Gegenstand der Erfindung ist die Verwendung einer Vorrichtung gemäß einem der Ansprüche 1 bis 8 zur Behandlung von Haaren.

Die so durchgeführte Avivagebehandlung des Haares ist denkbar einfach und kann beispielsweise ohne komplizierte Naßoperationen und das anschließende Trocknen durchgeführt werden. Auch kann das Avivageprodukt gezielt z. B. auf besonders splißgefährdeten Regionen aufgebracht werden.

Die Rezepturen der Wirkstoffzubereitungen können häufig auf wenige Bestandteile beschränkt werden, so daß auch hier Vorteile im Vergleich zu bisher üblichen Naßverfahren auftreten.

Die nachfolgenden Beispiele sollen den Erfindungsgegenstand näher erläutern ohne ihn jedoch hierauf zu beschränken.

## Beispiele

### 1. Rezepturen

Es wurden folgende Wirkstoffzubereitungen in einem Spender gemäß der deutschen Patentanmeldung 38 09 498 verwendet:

| Zubereitung | Komponenten | |
|---|---|---|
| A | Dehyquart(R) A[1] | 5 Gew.-% (bezogen auf Aktivsubstanz) |
| | Wasser | ad 100 Gew.-% |
| B | Dehyquart(R) E[2] | 5 Gew.-% (bezogen auf Aktivsubstanz) |
| | Wasser | ad 100 Gew.-% |
| C | Dehyton(R) AB 30[3] | 5 Gew.-% (bezogen auf Aktivsubstanz) |
| | N-Stearyl-N,N-dimethyl-ammoniumglycinat | 2,5 Gew.-% (bezogen auf Aktivsubstanz) |
| | Wasser | ad 100 Gew.-% |

D  Dehyquart[R] A     5 Gew.-% (bezogen auf Aktivsubstanz)

Phospholipone-100[4]    5 Gew.-% (bezogen auf Aktivsubstanz)

Wasser      ad 100 Gew.-%

E  Dehyquart[R] A     5 Gew.-% (bezogen auf Aktivsubstanz)

Cetiol[R] HE[5]     5 Gew.-% (bezogen auf Aktivsubstanz)

Wasser      ad 100 Gew.-%

1 Trimethylhexadecylammoniumchlorid (35 % Aktivsubstanz in Wasser) (HENKEL)

2 Dimethyl-N-(2-hydroxyethyl)-N-(2-hydroxyhexadecyl)-ammoniumchlorid (ca. 27 % Aktivsubstanz in Wasser) (HENKEL)

3 Fettamin-Derivat mit Betainstruktur, CTFA-Bezeichnung: Coco-Betaine (ca. 30 % Aktivsubstanz und 6 % NaCl in Wasser) (HENKEL)

4 Lecithin (NATTERMANN)

5 Polyolfettsäureester (HENKEL)

2. Bestimmung der Naß- und Trockenkämmbarkeit

Zur Bestimmung der Naß- und Trockenkämmbarkeit der mit der erfindungsgemäßen Vorrichtung behandelten Haare wurden 20 jeweils 0,8 g schwere, 11 cm lange braune europäische Haarsträhnen durch Behandlung mit einer Salzlösung, einer alkoholischen Lösung, und einer Aniontensidlösung definiert gereinigt. Sodann wurden die Haare mit 30 °C warmem Wasser gründlich ausgespült, das Wasser abgestreift und die Haare anschließend bei 30 °C und 40 % Luftfeuchtigkeit 5 Stunden lang getrocknet. Zur Bestimmung der Trockenkämmbarkeit wurden die Haare dann mit der erfindungsgemäßen Vorrichtung behandelt, in dem 20 Kammstriche durch das Haar durchgeführt wurden. Anschließend erfolgte die Messung des Kämmwiderstandes, d.h. der Kraft, die erforderlich war, um einen Kamm durch die Haarsträhnen zu ziehen. Zur Verringerung des Meßfehlers wurde die Bestimmung 15mal durchgeführt und der Mittelwert der Arbeitsintegrale gebildet. Die Messung erfolgte in einer Zug-Prüfmaschine, bei der die Haarsträhne mit zwei scherenförmig angeordneten Kämmen durchkämmt wird. Um zu vermeiden, daß die Strähne bei weiteren Kämmungen an der gleichen Stelle durchkämmt wird, kann das Kammsystem um eine Achse gedreht werden. Die Auswertung der Meßwerte erfolgte direkt über einen angeschlossenen Rechner.

Die Trockenkämmbarkeitsverbesserung (oder -verschlechterung) wurde definiert als

$$TK \; = \; \frac{\text{Arbeitsintegral bei behandelten Haaren}}{\text{Vergleichswert}} \cdot 100 \; \%$$

Als Vergleichwert wurde das Arbeitsintegral bei der Messung des Kämmwiderstandes der gereinigten, mit Wasser gespülten und getrockneten, nicht aber mit der erfindungsgemäßen Vorrichtung behandelten Haare bestimmt.

Zur Bestimmung der Naßkämmbarkeit wurden die Haare nach der analogen Behandlung mit der erfindungsgemäßen Vorrichtung definiert in einen nassen Zustand versetzt. Dies geschah in einer Apparatur, bestehend aus einer Wanne, einem Kamm-System und einem dünnen Rohr zur Wasserzufuhr. Das Kamm-System bestand aus 2 Kämmen, die um 180° versetzt mit dem Rücken an einer drehbaren Walze federnd befestigt waren und jeweils zur Hälfte einen großen und einen kleinen Zinkenabstand aufwiesen. Durch diese Anordnung wurden die Haarsträhnen im Verlauf einer Drehung der Walze jeweils einmal mit einem Kamm mit großem Zinkenabstand und einmal mit einem Kamm mit kleinem Zinkenabstand durchkämmt. Die Haare konnten so in einen definiert vorgeordneten, nassen Zustand versetzt werden. Nähere Einzelheiten zu dieser Anordnung sind einem Artikel der Erfinder in der Zeitschrift "Ärztliche Kosmetologie" zu entnehmen, der sich zur Zeit im Druck befindet.

Die Naßkämmbarkeitsverbesserung (oder -verschlechterung) wurde analog definiert als

$$NK \; = \; \frac{\text{Arbeitsintegral bei behandelten Haaren}}{\text{Vergleichswert}} \cdot 100 \; \%$$

Als Vergleichwert wurde das Arbeitsintegral bei der Messung des Kämmwiderstandes der gereinigten, mit Wasser gespülten, getrockneten und definiert in einen nassen Zustand versetzten, nicht aber mit der erfindungsgemäßen Vorrichtung behandelten Haare bestimmt.

Die Ergebnisse sind in der folgenden Tabelle zusammengefaßt:

| Zubereitung | TK [%] | NK [%] |
|---|---|---|
| A | 22 | 56 |
| B | 64 | 34 |
| C | 37 | 38 |
| D | 23 | 37 |
| E | 44 | 35 |

Die Ergebnisse zeigen die starke Naß- und Trockenkämmbarkeitsverbesserung der Haare, die durch die Verwendung der erfindungsgemäßen Vorrichtung erzielt wird.

Ansprüche

1. Vorrichtung zur Haarbehandlung, bestehend aus einem Spender in Form eines Kammes oder einer Bürste und einer Wirkstoffzubereitung, die mindestens eine haaravivierend oder haarfestigend wirkende Komponente enthält, dadurch gekennzeichnet, daß die haaravivierenden und/oder haarfestigenden Komponenten in einer Konzentration von 0,01 bis 20 Gew.-%, bezogen auf die Gesamtmasse der Wirkstoffzubereitung, enthalten sind und daß die Wirkstoffzubereitung bei Raumtemperatur eine Viskosität von 0,9 bis 12 mPas aufweist.

2. Vorrichtung nach Anspruch 1, dadurch gekennzeichnet, daß der Spender besteht aus einem Speicher für die Wirkstoffzubereitung aus einem ersten porösen Material innerhalb eines Gehäuses und in das erste poröse Material eingesetzten, mit diesem in Kapillarverbindung stehenden Zinken aus einem zweiten porösen Material, wobei die Zinken im Oberflächenbereich ihrer freien Enden mit verschlossenen Poren ausgebildet sind und die Hauptporenrichtung des zweiten porösen Materials der Zinken und des ersten porösen Materials des Speichers im wesentlichen gleichgerichtet parallel zur Zinkenlängsrichtung ist und sich das erste poröse Material des Speichers über ein Mehrfaches der Eindringtiefe der Zinken in Eindringrichtung erstreckt.

3. Vorrichtung nach Anspruch 1 oder 2, dadurch gekennzeichnet, daß die Wirkstoffzubereitung eine Oberflächenspannung von 20-70 dyn/cm, insbesondere von 30-40 dyn/cm, aufweist.

4. Vorrichtung nach einem der Ansprüche 1 bis 3, dadurch gekennzeichnet, daß die Wirkstoffzubereitung in Form einer wäßrigen Lösung oder Emulsion formuliert ist.

5. Vorrichtung nach einem der Ansprüche 1 bis 4, dadurch gekennzeichnet, daß die haaravivierende Komponente eine Löslichkeit von mindestens 0,1 g/l in Wasser aufweist.

6. Vorrichtung nach einem der Ansprüche 1 bis 5, dadurch gekennzeichnet, daß die Wirkstoffzubereitung als haaravivierende Komponente quartäre Ammoniumverbindungen enthält.

7. Vorrichtung nach einem der Ansprüche 1 bis 6, dadurch gekennzeichnet, daß die Wirkstoffzubereitung als haarfestigende Komponente filmbildende Polymere enthält.

8. Vorrichtung nach einem der Ansprüche 1 bis 7, dadurch gekennzeichnet, daß die Wirkstoffzubereitung ein organisches Lösungsmittel enthält.

9. Verwendung einer Vorrichtung nach einem der Ansprüche 1 bis 8 zur Behandlung von Haaren.